(19) Europäisches Patentamt
European Patent Office
Office européen des brevets

(11) **EP 2 229 098 B1**

(12) **EUROPÄISCHE PATENTSCHRIFT**

(45) Veröffentlichungstag und Bekanntmachung des Hinweises auf die Patenterteilung:
**16.03.2016 Patentblatt 2016/11**

(21) Anmeldenummer: **08866368.7**

(22) Anmeldetag: **08.12.2008**

(51) Int Cl.:
*A61B 5/06* (2006.01)   *A61B 5/00* (2006.01)
*A61B 1/273* (2006.01)   *A61B 5/07* (2006.01)
*A61B 90/00* (2016.01)   *A61B 34/30* (2016.01)
*A61B 34/20* (2016.01)   *A61B 1/00* (2006.01)
*A61B 1/04* (2006.01)

(86) Internationale Anmeldenummer:
**PCT/EP2008/066988**

(87) Internationale Veröffentlichungsnummer:
**WO 2009/083409 (09.07.2009 Gazette 2009/28)**

(54) **POSITIONSKONTROLLE MEDIZINISCHER GERÄTE IM MENSCHLICHEN KÖRPER MITTELS PHASENDIFFERENZMESSUNG**

POSITION CONTROL OF MEDICAL APPLIANCES IN THE HUMAN BODY BY MEANS OF PHASE DIFFERENCE MEASUREMENT

CONTRÔLE DE POSITION D'APPAREILS MÉDICAUX DANS LE CORPS HUMAIN AU MOYEN D'UNE MESURE DE DIFFÉRENCE DE PHASE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **02.01.2008 DE 102008003005**

(43) Veröffentlichungstag der Anmeldung:
**22.09.2010 Patentblatt 2010/38**

(73) Patentinhaber: **Siemens Aktiengesellschaft**
**80333 München (DE)**

(72) Erfinder:
• **DIEHL, Dirk**
**91052 Erlangen (DE)**
• **OPPELT, Ralph**
**91080 Uttenreuth (DE)**
• **REINSCHKE, Johannes**
**90419 Nürnberg (DE)**

(56) Entgegenhaltungen:
EP-A- 1 440 659   DE-A1-102006 019 415
DE-B3- 10 340 925   DE-C1- 10 142 253
US-A1- 2006 122 494

EP 2 229 098 B1

**Beschreibung**

Die Erfindung betrifft Vorrichtungen zur Beeinflussung der Position des medizinischen Gerätes.

Die Verwendung von Endoskopiekapseln findet in der Medizin zur Diagnose oder zur Behandlung des Inneren eines Patienten immer breitere Anwendung. Eine Endoskopiekapsel kann u.a. medizinische Instrumente bspw. zur Biopsie oder zum Einbringen von Medikamenten in den Körper und/oder bildgebende Systeme wie Kameras beinhalten. Darüber hinaus kann in der Kapsel ein Permanentmagnet integriert sein, der der Kapsel ein magnetisches Dipolmoment verleiht, so dass sie wie bspw. in der DE 103 40 925 B3 beschrieben mit Hilfe einer Magnetspulenanordnung beliebig manövrierbar ist.

Generell wird bei Untersuchungen des Körperinneren mit einem medizinischen Gerät wie einer Endoskopiekapsel die Position des Geräts überwacht und ggf. beeinflusst. Bspw. bei einer Untersuchung des Magens ist dieser halb mit Wasser gefüllt und die Endoskopiekapsel schwimmt an der Wasseroberfläche. Bei der Aufnahme von Bildern der Mageninnenwand tritt das Problem auf, dass die Kapsel und mit ihr die Kamera aufgrund der nicht zu vermeidenden Wasserbewegung in Bewegung versetzt wird, so dass lediglich unscharfe, verwackelte Bilder aufgenommen werden können. Auch für den Fall, dass eine Serie von Bildern eines bestimmten Bereichs aufgenommen werden soll, ist es notwendig, dass die Kapsel still steht.

Zur Positionsbestimmung nutzen elektromagnetische Messverfahren meist niederfrequente magnetische Wechselfelder, die den menschlichen Körper nahezu unbeeinflusst durchdringen, womit eine absolute Positionsbestimmung möglich wird. Ein derartiges System ist in WO 2005 / 120345 A2 beschrieben. Bekannte Systeme haben jedoch zum Einen den Nachteil einer begrenzten Messgenauigkeit. Zum Anderen ist bedingt durch ein schlechtes Signal-zu-Rausch-Verhältnis und der damit verbundenen notwendigen großen Integrationszeit die zeitlichen Auflösung relativ gering und die Messwertverzögerung vergleichsweise groß. Alternativ wurden zur absoluten Positionsmessung medizinischer Geräte im Körperinneren Phasendifferenzmessungen an hochfrequenten elektromagnetischen Wellen vorgeschlagen. Dabei wurde nicht berücksichtigt, dass die Wellenausbreitung durch Körpergewebe mit unterschiedlicher Dielektrizitätszahl und Leitfähigkeit zu einer erheblichen Deformation der im Freiraum kugelförmigen Wellenfronten führen. Um trotzdem eine absolute Positionsbestimmung zu ermöglichen, sind aufwändige Korrekturverfahren notwendig.

Die EP 1 440 659 A2 offenbart die Merkmale des Oberbegriffs von Anspruch 1 bzw. Anspruch 6 und beschreibt ein Positionsmesssystem mit einer in-vivo Funkeinrichtung und einer Vielzahl von ex-vivo Funkeinrichtungen. Jede der ex-vivo Funkeinrichtungen enthält einen Sender zum Übertragen eines Positionssignals und die in-vivo Funkeinrichtung umfasst einen Empfänger zum Empfangen des Positionssignals. Zudem umfasst die in-vivo Funkeinrichtung eine Positionsmesseinheit, die dazu ausgebildet ist, die Position der in-vivo Funkeinrichtung anhand der Positionssignale zu bestimmen, die von den ex-vivo Funkeinrichtungen empfangen werden.

Des Weiteren beschreibt die DE 10 2006 019 415 A1 eine Vorrichtung zur Darstellung der Position einer medizinischen Einrichtung im Körper eines Lebewesens. Die medizinische Einrichtung umfasst einen Transponder. Zudem umfasst die Vorrichtung einen Sender, der außerhalb des Körpers angeordnet ist, zum Senden eines Sendesignals zu dem Transponder und eine Empfänger, der außerhalb des Körpers angeordnet ist, zum Empfangen eines von dem Transponder in Folge des Sendesignals abgestrahlten Reflexionssignals. Des Weiteren umfasst die Vorrichtung eine Positionsermittlungseinrichtung zum Ermitteln der Position des Transponders anhand einer Phasendifferenz zwischen dem Sendesignal und dem Reflexionssignal.

Aufgabe der Erfindung ist es daher, eine Vorrichtung anzugeben, mit dem eine Positionsänderung eines medizinischen Gerätes detektiert und einer derartigen Änderung entgegengewirkt werden kann.

Diese Aufgabe wird durch die in den unabhängigen Ansprüchen angegebenen Erfindungen gelöst. Vorteilhafte Ausgestaltungen ergeben sich aus den abhängigen Ansprüchen.

Die vorliegende Erfindung geht davon aus, dass zur Kontrolle der Position eines medizinischen Gerätes im Körperinneren und für eine eventuelle Positionskorrektur nicht die absolute Position des Gerätes benötigt wird, sondern lediglich Positionsänderungen nach ihrer Richtung und zumindest grob nach ihrem Betrag detektiert werden müssen. Beim Feststellen einer Abweichung des medizinischen Gerätes von einer Sollposition oder allgemeiner wenn das medizinische Gerät eine ungewollte Bewegung durchführt, kann eine Regelung eingesetzt werden, die der Abweichung bzw. der Bewegung entgegenwirkt. Es reicht demnach aus, lediglich eine relative Positionsbestimmung durchzuführen.

Hierzu sendet das medizinische Gerät kontinuierlich oder in Intervallen hochfrequente elektromagnetische Signale aus, die von mehreren räumlich verteilten Empfangseinrichtungen empfangen werden. Zur Detektion einer Bewegung des medizinischen Gerätes wird das zeitliche Verhalten der Phasendifferenzen zwischen den an den Empfangseinrichtungen empfangenen Signalen und einem Referenzsignal überwacht. Das Referenzsignal kann dabei von einer separaten Referenzsignalquelle oder von einer der Empfangseinrichtungen stammen. Für den Fall, dass eine oder mehrere der Phasendifferenzen der Empfangseinrichtungen sich verändern, ist davon auszugehen, dass sich das medizinische Gerät bewegt hat, so dass ggf. entsprechende Gegenmaßnahmen getroffen werden können, um der Bewegung entgegenzuwirken.

Die Gegenmaßnahmen werden von einer Regeleinrichtung in Abhängigkeit von den detektierten Phasendifferenzen ausgelöst. Die Regeleinrichtung regelt eine Manövriervorrichtung zur Beeinflussung der Position des medizinischen Gerätes, wobei die Manövriervorrichtung eine Magnetspulenanordnung sein kann, wie sie in DE 103 40 925 B3 beschrieben wird.

Die erfindungsgemäße Vorrichtung bringt dadurch, dass lediglich eine relative Positionsmessung durchgeführt wird, den Vorteil mit sich, dass wegen des hohen Signal-zu-Rausch-Verhältnisses eine schnelle Messung und damit eine kurze Reaktionszeit gegeben ist. Abweichungen des medizinischen Gerätes von einer Sollposition werden daher schnell detektiert und können entsprechend kurzfristig korrigiert werden, bevor der Betrag der Positionsänderung zu groß wird. Darüber hinaus werden im Unterschied zu absoluten Positionsmessungen vorteilhafterweise keine Kenntnisse über das zwischen dem medizinischen Gerät bzw. der Sendeeinrichtung und den Empfangseinrichtungen befindliche Körpergewebe (bspw. Dielektrizitätszahl, Leitfähigkeit) benötigt.

Um eine genauere und schnellere absolute Positionsmessung zu ermöglichen, ist es denkbar, die erfindungsgemäße Vorrichtung mit anderen, z.B. niederfrequenten Messverfahren zur absoluten Positionsmessung zu kombinieren.

Weitere Vorteile, Merkmale und Einzelheiten der Erfindung ergeben sich aus dem im Folgenden beschriebenen Ausführungsbeispiel sowie anhand der Zeichnungen.

Dabei zeigt:

Figur 1    ein erstes Ausführungsbeispiel der erfindungsgemäßen Vorrichtung,
Figur 2    ein zweites Ausführungsbeispiel der erfindungsgemäßen Vorrichtung,
Figur 3    eine Anordnung einer Vielzahl von Empfangseinrichtungen und eines medizinischen Gerätes an einem Patienten,
Figur 4    eine Übersicht über die sich bei einer Bewegung eines medizinischen Gerätes gemäß Figur 3 ergebenden Änderungen der Phasendifferenzen und
Figur 5    eine Manövriervorrichtung.

Erstes Ausführungsbeispiel

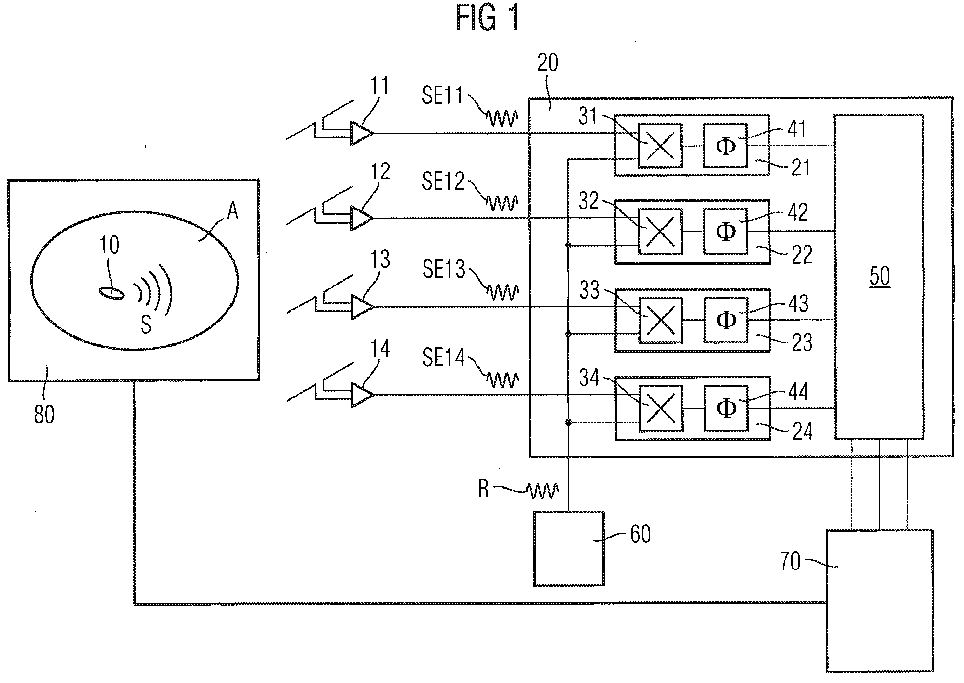

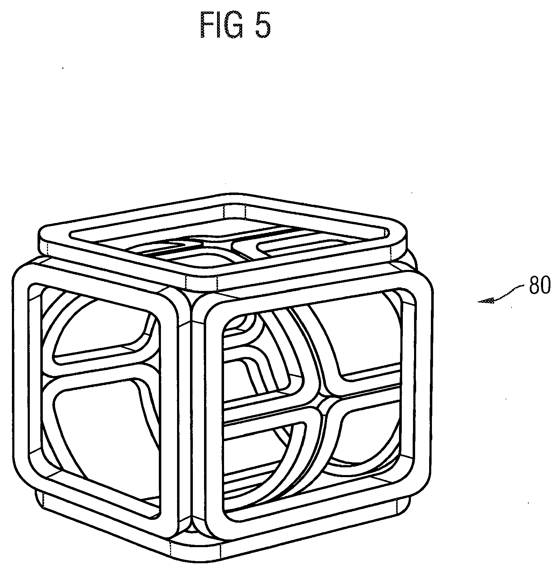

Die Figur 1 zeigt eine erfindungsgemäße Vorrichtung zur Kontrolle einer Position x,y,z eines medizinischen Gerätes 10 in einem Arbeitsraum A in einer ersten Ausführungsform. Der Arbeitsraum A kann dabei ein Hohlraum im Inneren eines Patienten wie bspw. der Magen sein, während das medizinische Gerät 10 vorzugsweise eine Endoskopiekapsel ist. Die Endoskopiekapsel 10 ist mit einem Permanentmagneten ausgestattet und weist daher ein magnetisches Dipolmoment auf, so dass sie mit Hilfe einer Manövriervorrichtung 80 bzw. Magnetspulenanordnung wie bspw. in der DE 103 40 925 B3 beschrieben und entsprechend in der Figur 5 exemplarisch dargestellt magnetisch und berührungslos manövrierbar ist.

Darüber hinaus enthält die Endoskopiekapsel 10 eine Sendeeinrichtung. Diese sendet fortwährend oder in Intervallen ein moduliertes oder unmoduliertes Signal S, bspw. ein Hochfrequenzsignal S mit einer Frequenz von 435MHz.

Das Signal S wird von einer oder mehreren, im ersten Ausführungsbeispiel von vier Empfangseinrichtungen 11-14 empfangen. Hierzu sind die Empfangseinrichtungen 11-14 mit einer Antenne zum Empfangen eines elektrischen und/oder eines magnetischen Feldes ausgestattet. Darüber hinaus enthalten die Empfangseinrichtungen 11-14 jeweils einen Vorverstärker zum Verstärken des empfangenen Signals. Die mit den Empfangseinrichtungen 11-14 empfangenen und verstärkten Signale SE11-SE14 werden an eine Signalverarbeitungseinrichtungen 20 übertragen. Die Signalverarbeitungseinrichtung 20 enthält mehrere Einrichtungen 21-24, wobei jeder Empfangseinrichtung 11-14 eine Einrichtung 21-24 zugeordnet ist. Die Einrichtungen 21-24 verfügen jeweils über einen ersten und einen zweiten Signaleingang sowie über einen Signalausgang, wobei die empfangenen Signale SE11-SE14 jeweils am zweiten Signaleingang anliegen.

Darüber hinaus ist eine Referenzsignalquelle 60 vorgesehen, die ein Referenzsignal R erzeugt. Die Referenzsignalquelle 60 kann ein Referenzoszillator sein, dessen Frequenz vorzugsweise nur geringfügig von der Frequenz des Signals S abweicht. Das Referenzsignal R liegt an jedem ersten Signaleingang der Einrichtungen 21-24 an.

Die Einrichtungen 21-24 enthalten vorzugsweise jeweils einen Mischer 31-34 und einen Phasenmesser 41-44, wobei jeder Mischer 31-34 jeweils einen ersten und einen zweiten Signaleingang aufweist. Die ersten bzw. zweiten Signaleingänge der Einrichtungen 21-24 entsprechen den ersten bzw. zweiten Eingängen der Mischer 31-34. Die Signalausgänge der Phasenmesser 41-44 entsprechen den Signalausgängen der Einrichtungen 21-24.

Die an den Signaleingängen eines Mischers 31-34 anliegenden Signale werden in an sich bekannter Weise miteinander heruntergemischt. Die Ausgangssignale der Mischer 31-34 werden jeweils an einen Signaleingang der Phasenmesser 41-44 weitergeleitet. Der Phasenmesser 41-44 bestimmt die Phasenlage des an seinem Eingang anliegenden Signals, wobei bspw. das Signal zunächst derart verstärkt wird, dass näherungsweise ein Rechtecksignal entsteht, und anschließend der Nulldurchgang des Rechtecksignals bestimmt wird. Die Ausgangssignale der Phasenmesser 41-44 entsprechen dann jeweils den Phasenabweichungen $d\varphi_{11}$, $d\varphi_{12}$, $d\varphi_{13}$, $d\varphi_{14}$ zwischen den Phasen der Signale, die an den ersten und zweiten Signaleingängen der Einrichtungen 21-24 bzw. der Mischer 31-34 anliegen: Bspw. liegt am ersten Signaleingang der Einrichtung 21 das Signal R an, während am zweiten Signaleingang der Einrichtung 21 das an der Empfangseinrichtung 11 empfangene Signal SE11 anliegt. Das Ausgangssignal der Einrichtung 21 entspricht dann der Abweichung $d\varphi_{11} = \varphi(SE11)-\varphi(R)$ zwischen der Phase $\varphi(SE11)$ des Signals SE11 und der Phase $\varphi(R)$ des Referenzsignals R. Entsprechendes gilt für die Eingangs- und Ausgangssignale der Einrichtungen 22-24, d.h. die Ausgangssignale der Einrichtungen 21-24 entsprechen den Phasenabweichungen $d\varphi_{11}$, $d\varphi_{12}$, $d\varphi_{13}$, $d\varphi_{14}$ zwischen den Phasen $\varphi(SE11)$, $\varphi(SE12)$, $\varphi(SE13)$, $\varphi(SE14)$ der an den Empfangseinrichtungen 11-14 empfangenen Signalen SE11-SE14 und der Phase $\varphi(R)$ des von der Referenzsignalquelle 60 erzeugten Referenzsignals R.

Da die Frequenzen der Referenzsignalquelle 60 und der Sendeeinrichtung der Endoskopiekapsel 10 in der Regel nicht exakt übereinstimmen, sind die Phasenabweichungen $d\varphi_{11}$, $d\varphi_{12}$, $d\varphi_{13}$, $d\varphi_{14}$ nicht zeitlich konstant sondern wachsen linear mit der Zeit. Unter der Voraussetzung, dass die Endoskopiekapsel 10 nicht bewegt wird, muss jedoch die Differenz zwischen den Abweichungen zeitlich konstant sein. In der Signalverarbeitungseinrichtung 20 ist daher eine Differenzbildungsvorrichtung 50 vorgesehen, in die die Abweichungen $d\varphi_{11}$, $d\varphi_{12}$, $d\varphi_{13}$, $d\varphi_{14}$ eingespeist werden.

In der Differenzbildungsvorrichtung 50 werden Phasendifferenzen $\Delta\varphi_1$, $\Delta\varphi_2$, $\Delta\varphi_3$ ermittelt. Dabei wird eine beliebige der Phasenabweichungen $d\varphi_{11}$, $d\varphi_{12}$, $d\varphi_{13}$, $d\varphi_{14}$ als Referenzwert $d\varphi_{ref}$ festgelegt, bspw. $d\varphi_{ref} = d\varphi_{11}$, und die Differenz zwischen den übrigen Phasenabweichungen $d\varphi_{12}$, $d\varphi_{13}$, $d\varphi_{14}$ und dem Referenzwert $d\varphi_{ref}$ gebildet, d.h. $\Delta\varphi_1 = d\varphi_{12} - d\varphi_{11}$, $\Delta\varphi_2 = d\varphi_{13} - d\varphi_{11}$ und $\Delta\varphi_3 = d\varphi_{14} - d\varphi_{11}$. Die Auswahl einer der Abweichungen als Referenzwert $d\varphi_{ref}$ kann willkürlich erfolgen oder bspw. in Abhängigkeit vom Betrag der Abweichungen $d\varphi_{11}$, $d\varphi_{12}$, $d\varphi_{13}$, $d\varphi_{14}$.

Die Phasendifferenzen $\Delta\varphi_1$, $\Delta\varphi_2$, $\Delta\varphi_3$ werden zeitlich fortlaufend oder in Intervallen bestimmt.

Die Phasendifferenzen $\Delta\varphi_1$, $\Delta\varphi_2$, $\Delta\varphi_3$ werden einer Regeleinrichtung 70 zugeführt. Die Regeleinrichtung 70 ist mit einer Manövriervorrichtung 80 zur Beeinflussung der Position x,y,z der Endoskopiekapsel 10 verbunden und verwendet die Phasendifferenzen $\Delta\varphi_1$, $\Delta\varphi_2$, $\Delta\varphi_3$ zur Regelung der Manövriervorrichtung 80. Dabei definiert x,y,z die Position des Schwerpunktes der Endoskopiekapsel 10 in einem kartesischen Koordinatensystem, welches bspw. durch die Geometrie der Manövriervorrichtung 80 vorgegeben sein kann.

Zweites Ausführungsbeispiel

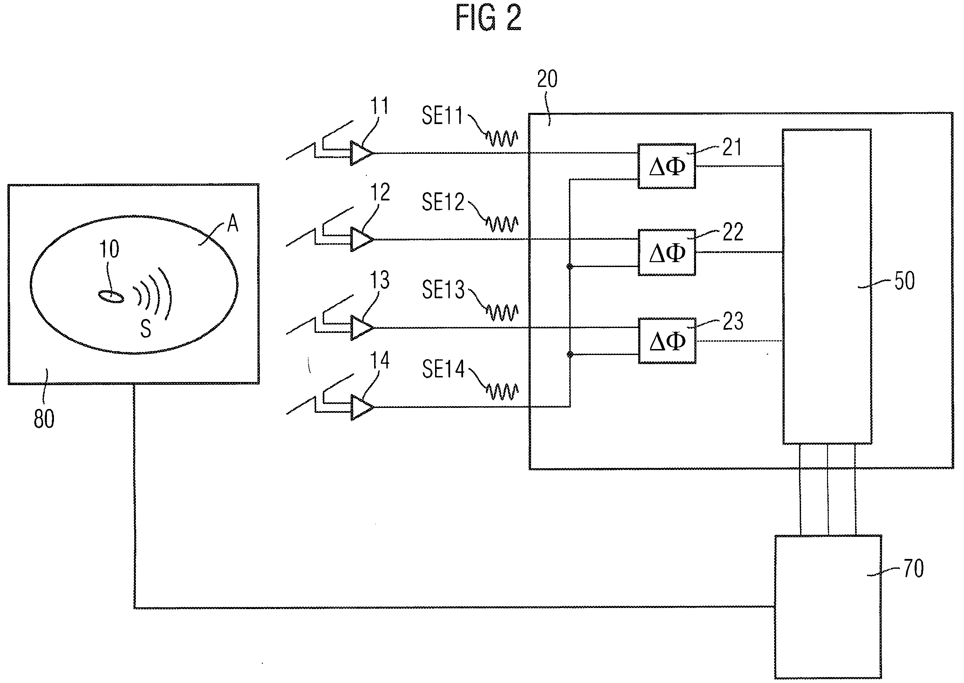

In einem zweiten, bevorzugten Ausführungsbeispiel, das in der Figur 2 dargestellt ist, sendet das medizinische Gerät 10 wie im ersten Ausführungsbeispiel mit Hilfe einer Sendeeinrichtung fortwährend oder in Intervallen ein moduliertes oder unmoduliertes Signal S. Das Signal S wird von Empfangseinrichtungen 11-14 empfangen, wobei im Folgenden eine der Empfangseinrichtungen 11-14 als erste Empfangseinrichtung 14 und die übrigen Empfangseinrichtungen als zweite Empfangseinrichtungen 11-13 bezeichnet werden. Die Empfangseinrichtungen 11-14 umfassen jeweils eine Antenne zum Empfangen eines elektrischen und/oder eines magnetischen Feldes sowie einen Vorverstärker zum Verstärken des empfangenen Signals.

Die mit den Empfangseinrichtungen 11-14 empfangenen und verstärkten Signale SE11-SE14 werden an eine Signalverarbeitungseinrichtung 20 übertragen. In der Signalverarbeitungseinrichtung 20 werden Phasendifferenzen $\Delta\varphi_1$, $\Delta\varphi_2$, $\Delta\varphi_3$ zwischen den an den zweiten Empfangseinrichtungen 11-13 empfangenen Signalen SE11-SE13 und dem an der ersten Empfangseinrichtung 14 empfangenen Signal SE14 ermittelt, d.h.

$$\Delta\varphi_1 = \varphi(SE11) - \varphi(SE14), \quad \Delta\varphi_2 = \varphi(SE12) - \varphi(SE14),$$

$$\Delta\varphi_3 = \varphi(SE13) - \varphi(SE14), \text{ wobei } \varphi(X) \text{ die Phase eines Signals X}$$

wobei $\varphi(X)$ die Phase eines Signals X symbolisiert. Das empfangene Signal SE14 der ersten Empfangseinrichtung 14 dient demnach im Wortsinn des ersten Ausführungsbeispiels als Referenzsignal R.

Zur Ermittlung der Phasendifferenzen $\Delta\varphi_1$, $\Delta\varphi_2$, $\Delta\varphi_3$ sind Einrichtungen 21-23, bspw. Phasendetektoren 21-23 vorgesehen, wobei die Anzahl der Phasendetektoren 21-23 mindestens der Anzahl der zweiten Empfangseinrichtungen 11-13 entspricht. Jeder Phasendetektor 21-23 weist einen ersten und einen zweiten Signaleingang sowie einen Signalausgang auf. Dabei ist die erste Empfangseinrichtung 14 zum Übertragen des empfangenen Signals SE14 mit dem ersten Signaleingang jedes Phasendetektors 21-23 verbunden. Die zweiten Empfangseinrichtungen 11-13 sind jeweils mit dem zweiten Signaleingang der Phasendetektoren 21-23 verbunden, während die Signalausgänge zum Übertragen der ermittelten Phasendifferenzen $\Delta\varphi_1$, $\Delta\varphi_2$, $\Delta\varphi_3$ mit einer Regeleinrichtung 70 verbunden sind.

Da die Frequenzen der empfangenen Signale SE11-SE14 identisch sind, können im zweiten Ausführungsbeispiel die Phasendifferenzen im Unterschied zum ersten Ausführungsbeispiel direkt bestimmt werden.

Die Phasendifferenzen $\Delta\varphi_1$, $\Delta\varphi_2$, $\Delta\varphi_3$ werden wie im ersten Ausführungsbeispiel der Regeleinrichtung 70 zugeführt. Die Regeleinrichtung 70 ist wie im ersten Ausführungsbeispiel mit einer Manövriervorrichtung 80 zur Beeinflussung der Position x,y,z der Endoskopiekapsel 10 verbunden und verwendet die Phasendifferenzen $\Delta\varphi_1$, $\Delta\varphi_2$, $\Delta\varphi_3$ zur Regelung der Manövriervorrichtung 80.

Die Signalverarbeitungseinrichtung 20 ist derart eingerichtet, dass anstelle des empfangenen Signals SE14 der ersten Empfangseinrichtung 14 ein an einer beliebigen der anderen Empfangseinrichtungen 11-13 empfangenes Signal SE11-SE13 als Referenzsignal R verwendet werden kann, d.h. bspw. das Signal SE12 der Empfangseinrichtung 12. Dementsprechend würden die Phasendifferenzen $\Delta\varphi_1$, $\Delta\varphi_2$, $\Delta\varphi_3$ berechnet gemäß

$$\Delta\varphi_1 = \varphi(SE11) - \varphi(SE12), \quad \Delta\varphi_2 = \varphi(SE13) - \varphi(SE12),$$

$$\Delta\varphi_3 = \varphi(SE14) - \varphi(SE12). \text{ Die Empfangseinrichtung 12 übernimmt}$$

Die Empfangseinrichtung 12 übernimmt dann die Rolle der ersten Empfangseinrichtung, während die Empfangseinrichtungen 11, 13, 14 die Gruppe der zweiten Empfangseinrichtungen bilden. Denkbar wäre eine Realisierung mit Hilfe eines ersten und eines zweiten Multiplexers, wobei der ersten Multiplexer aus den Signalen SE11-SE14 eines, z.B. SE14, auswählt und dieses an die ersten Signaleingänge der

Phasendetektoren 21-23 ausgibt, während der zweite Multiplexer aus den Signalen SE11-SE14 die übrigen drei Signale, im Beispiel SE11, SE12 und SE13, auswählt und diese jeweils an die zweiten Signaleingänge weiterleitet. Alternativ sind jedoch auch andere Möglichkeiten denkbar, eine beliebige der Empfangseinrichtungen 11-14 schaltungstechnisch als erste Empfangseinrichtung festzulegen und die Signale SE11-SE14 entsprechend an die ersten und zweiten Signaleingänge der Phasendetektoren zu leiten.

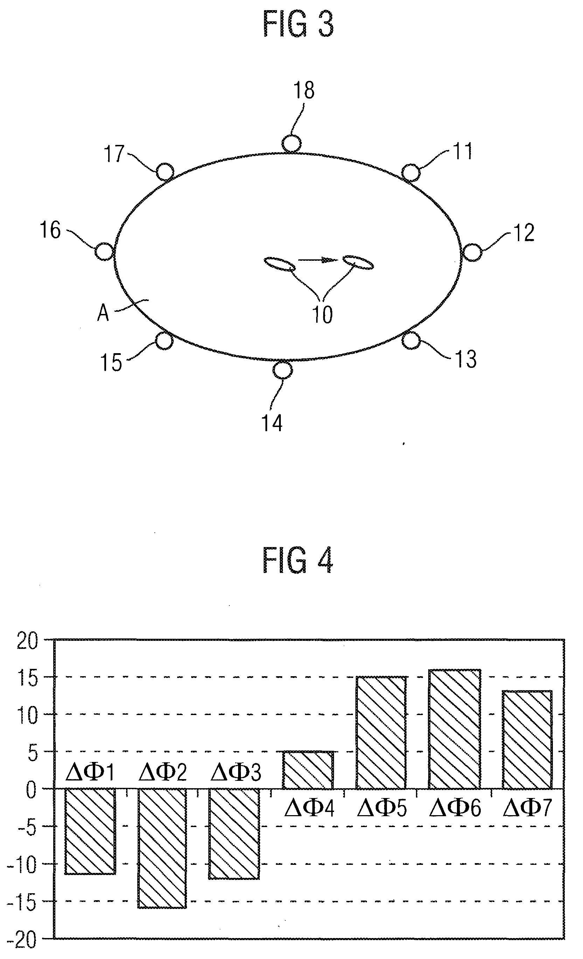

Vorteilhafterweise werden zur Erhöhung der Messgenauigkeit mehr als vier Empfangseinrichtungen verwendet. Die Figur 3 zeigt ein System bestehend aus einer Endoskopiekapsel 10 und acht Empfangseinrichtungen 11-18, die im Bereich eines Arbeitsraums A angebracht sind. In einer konkreten Anwendung kann der Arbeitsraum A das Innere eines Patienten sein, wobei sich die Endoskopiekapsel bspw. im Magen des Patienten befinden kann. Zusätzlich zu den in der Figur 3 dargestellten Empfangseinrichtungen 11-18 können weitere Empfangseinrichtungen in Ebenen vor und hinter der dargestellten Bildebene vorgesehen sein. Vorteilhafterweise sind die Empfangseinrichtungen derart angeordnet, dass der gesamte mit der Endoskopiekapsel 10 zu untersuchende Bereich von einem Netz von Empfangseinrichtungen umgeben ist.

Funktionsweise

Das erste und das zweite Ausführungsbeispiel unterscheiden sich in der Bereitstellung des Referenzsignals R. Während im ersten Ausführungsbeispiel eine separate Referenzsignalquelle 60 das Referenzsignal R bereitstellt, dient im zweiten Ausführungsbeispiel eine beliebige der Empfangseinrichtungen 11-14 als Quelle des Referenzsignals R. Die grundsätzlichen Verfahrensschritte des in der Regeleinrichtung 70 ablaufenden Verfahrens zur Kontrolle der Position x,y,z der Endoskopiekapsel 10 basierend auf den ermittelten Phasendifferenzen $\Delta\varphi_1$, $\Delta\varphi_2$, $\Delta\varphi_3$ sind für beide Ausführungsbeispiele identisch.

Mit dem in der Figur 3 dargestellten System werden sieben Phasendifferenzen $\Delta\varphi_1$ bis $\Delta\varphi_7$ ermittelt und der Regeleinrichtung 70 zugeführt. Für den Fall, dass die Endoskopiekapsel 10 nicht bewegt wird, d.h. relativ zu den Empfangseinrichtungen 11-18 still steht, sind die Phasendifferenzen $\Delta\varphi_1$ bis $\Delta\varphi_7$ zeitlich konstant.

Wenn die Kapsel 10 bewegt wird, ändern sich zumindest einige der Phasendifferenzen $\Delta\varphi_1$ bis $\Delta\varphi_7$ während der Bewegung. Dabei kann generell angenommen werden, dass eine große Änderung einer Phasendifferenz einhergeht mit einer großen Bewegung der Kapsel 10 in Richtung der Verbindungslinie zwischen der Kapsel 10 und der die entsprechenden Empfangseinrichtung.

Die Regeleinrichtung 70 wertet die ermittelten Phasendifferenzen $\Delta\varphi_1$ bis $\Delta\varphi_7$ aus, indem das zeitliche Verhalten $\Delta\varphi_1(t)$ bis $\Delta\varphi_7(t)$ der ihr zugeführten Phasendifferenzen $\Delta\varphi_1$ bis $\Delta\varphi_7$ überwacht wird. Dabei werden fortlaufend die momentanen, d.h. zu einem Zeitpunkt t2 ermittelten Phasendifferenzen $\Delta\varphi_1(t2)$ bis $\Delta\varphi_7(t2)$ mit den unmittelbar zuvor, zu einem Zeitpunkt t1 ermittelten Phasendifferenzen $\Delta\varphi_1(t1)$ bis $\Delta\varphi_7(t1)$ verglichen (t1 < t2).

Alternativ können zu einem ersten, beliebigen Zeitpunkt t1 die momentan anliegenden Phasendifferenzen $\Delta\varphi_1(t1)$ bis $\Delta\varphi_7(t1)$ als Referenzwerte gespeichert werden. Bspw. wenn ein Bediener des Systems die Endoskopiekapsel 10 in eine Sollposition x(t1),y(t1),z(t1) gebracht hat, in der eine Serie von Bildern eines bestimmten Bereichs der Mageninnenwand aufgenommen werden soll, ist es notwendig, dass die Kapsel 10 still steht. Zu diesem Zeitpunkt t1 werden bspw. auf Knopfdruck durch den Bediener die momentan ermittelten Phasendifferenzen $\Delta\varphi_1(t1)$ bis $\Delta\varphi_7(t1)$ gespeichert. Die späteren, zweiten Zeitpunkten t ermittelten Phasendifferenzen $\Delta\varphi_1(t)$ bis $\Delta\varphi_7(t)$ werden in der Regeleinrichtung 70 mit den gespeicherten Referenzwerten $\Delta\varphi_1(t1)$ bis $\Delta\varphi_7(t1)$ fortlaufend verglichen.

Bei einer Veränderung einer oder mehrerer der Phasendifferenzen $\Delta\varphi_1$ bis $\Delta\varphi_7$ wird eine Regelung der Manövriervorrichtung 80 durch die Regeleinrichtung 70 initiiert. Die Manövriervorrichtung 80 ist in den beiden Ausführungsbeispielen vorzugsweise eine Anordnung mehrerer Einzelspulen zur berührungslosen Führung der Endoskopiekapsel 10, wie sie bspw. als "Magnetspulenanordnung" in der DE 103 40 925 B3 beschrieben wird. Die Manövriervorrichtung 80 erzeugt durch entsprechende gezielte Bestromung der Einzelspulen eine oder mehrere Magnetfeldkomponenten $B_x$, $B_y$, $B_z$ und/oder eines oder mehrere Gradientenfelder $G_{ij} = \partial B_i / \partial j$ mit i, j = x, y, z, womit durch das Zusammenwirken mit dem magnetischen Dipolmoment des Permanentmagneten der Kapsel 10 Drehmomente und/oder Kräfte auf die Kapsel 10 ausgeübt werden können. Die gezielte Bestromung der Einzelspulen und in der Folge davon die Gradientenfelder $G_{ij}$ und/oder die Magnetfeldkomponenten $B_x$, $B_y$, $B_z$ werden in Abhängigkeit von der durch die Regeleinrichtung 70 vorgegebenen Regelung aufgebaut.

Die Regelung erfolgt dabei in der Weise, dass bei einer Änderung der Position x,y,z der Endoskopiekapsel 10, die wie oben beschrieben mit einer Änderung einer oder mehrerer Phasendifferenzen verbunden ist, die Gradientenfelder $G_{ij}$ und/oder die Magnetfeldkomponenten $B_x$, $B_y$, $B_z$ so eingestellt werden, dass die erzeugten Kräfte und Drehmomente der detektierten Bewegung der Kapsel entgegenwirken.

Da die Zusammenhänge zwischen der Bestromung einer oder mehrerer der Einzelspulen und den so erzeugbaren Drehmomenten und Kräften hinsichtlich Betrag und Richtung bekannt sind, kann der über die Überwachung der Phasendifferenzen detektierten Bewegung der Endoskopiekapsel gezielt entgegengewirkt werden, indem je nach detektierter Bewegungsrichtung und ggf. -amplitude die entsprechenden Einzelspulen bestromt werden. Zur grundsätzlichen Wirkungsweise der Manövriervorrichtung 80 wird auf DE 103 40 925 B3 verwiesen. Die Manövriervorrichtung 80 der erfindungsgemäßen Vorrichtung arbeitet in vergleichbarer Weise, ist jedoch nicht auf das Design der "Magnetspulenanordnung" der DE 103 40 925 B3 festgelegt sondern kann auch mehr oder weniger Einzelspulen umfassen und ggf. ausgebildet sein, um eine andere Anzahl magnetischer Freiheitsgrade zu erzeugen als die Manövriervorrichtung bzw. "Magnetspulenanordnung" der DE 103 40 925 B3.

Die Folge der Regelung der Manövriervorrichtung 80 durch die Regeleinrichtung 70 ist demnach, dass die Phasendifferenzen $\Delta\varphi_1(t)$ bis $\Delta\varphi_7(t)$ zeitlich konstant bleiben oder dass die momentan ermittelten Phasendifferenzen $\Delta\varphi_1(t)$ bis $\Delta\varphi_7(t)$ mit den gespeicherten Referenzwerten $\Delta\varphi_1(t1)$ bis $\Delta\varphi_7(t1)$ übereinstimmen. Ungewollten Bewegungen der Endoskopiekapsel oder Abweichungen der Position x,y,z der Kapsel 10 von einer Sollposition x(t1),y(t1),z(t1) kann so entgegengewirkt werden.

Weitere Ausführungen

Die in der Figur 3 durch den Pfeil angedeutete Bewegung der Kapsel 10 in x-Richtung schlägt sich in einer vergleichsweise großen Änderung der bzgl. der Empfangseinrichtungen 12, 16 ermittelten Phasendifferenzen $\Delta\varphi_2$, $\Delta\varphi_6$ nieder. Die hinsichtlich der Empfangseinrichtungen 14, 18 ermittelten Phasendifferenzen $\Delta\varphi_4$, $\Delta\varphi_8$ werden sich dagegen gar nicht oder nur minimal verändern. Die Figur 4 zeigt ein Diagramm, in dem für die Empfangseinrichtungen 11-18 die Änderungen der Phasendifferenzen $\Delta\varphi_1$ bis $\Delta\varphi_7$ in beliebigen Einheiten aufgetragen sind, die sich bei einer Bewegung der Kapsel 10 in x-Richtung gemäß Figur 3 ergeben können.

Vorzugsweise wird bei der Auswertung der Phasendifferenzen in der Regeleinrichtung 70 nur eine begrenzte Anzahl von Phasendifferenzen, insbesondere nur die betragsmäßig größten Phasendifferenzen $\Delta\varphi_1$, $\Delta\varphi_2$, $\Delta\varphi_3$, $\Delta\varphi_5$, $\Delta\varphi_6$, $\Delta\varphi_7$, berücksichtigt, während die übrigen $\Delta\varphi_4$ vernachlässigt wird. Alternativ kann eine Gewichtung entsprechend der Beträge der Phasendifferenzen erfolgen.

Für den Fall, dass die Endoskopiekapsel mit einem bildgebenden System wie einer Kamera ausgestattet ist und ein Videosignal übermittelt, kann der in der Kapsel zu diesem Zweck vorhandene Sender auch zur Übermittlung des Signals S verwendet werden, wobei typischerweise eine Trägerfrequenz von 433MHz eingesetzt wird. Auf eine separate Sendeeinrichtung oder andere zusätzliche Einbauten in der Kapsel zur Übertragung des Signals S zur Positionskontrolle kann dann verzichtet werden. Evtl. muss dafür das Sendeprogramm der Kapsel dahingehend verändert werden, dass die Bildübertragung in vorgegebenen Intervallen unterbrochen und für wenige microsec ein unmoduliertes Signal für die Positionsmessung gesendet wird.

Zudem werden bei einer Manövriervorrichtung 80 bzw. einer Magnetspulenanordnung wie sie in DE 103 40 925 B3 beschrieben wird die Empfangseinrichtungen im Innenraum der zylinderförmigen Manövriervorrichtung 80 bspw. an der Zylinderinnenwand, angebracht.

**Patentansprüche**

1. Vorrichtung zur Kontrolle einer Position (x,y,z) eines medizinischen Gerätes (10) in einem Arbeitsraum (A), wobei das medizinische Gerät (10) eine Sendeeinrichtung zum Senden eines Signals (S) umfasst, mit

   - einer oder mehreren Empfangseinrichtungen (11-14) zum Empfangen des Signals (S),
   - einer Referenzsignalquelle (60) zum Erzeugen eines Referenzsignals (R) und
   - einer Signalverarbeitungseinrichtung (20) zum Ermitteln von Phasendifferenzen ($\Delta\varphi_1$, $\Delta\varphi_2$, $\Delta\varphi_3$, ... ) basierend auf den an den Empfangseinrichtungen (11-14) empfangenen Signalen (SE11-SE14) und dem Referenzsignal (R),

   wobei

   - die Empfangseinrichtungen (11-14) und die Referenzsignalquelle (60) zum Übertragen der empfangenen Signale (SE11-SE14) und des Referenzsignals (R) mit der Signalverarbeitungseinrichtung (20) verbunden sind,
   - einer Regeleinrichtung (70) und
   - einer Manövriervorrichtung (80), wobei
   - die Signalverarbeitungseinrichtung (20) zum Übertragen der Phasendifferenzen ($\Delta\varphi_1$, $\Delta\varphi_2$, $\Delta\varphi_3$, ... ) mit der Regeleinrichtung (70) verbunden ist und
   - die Regeleinrichtung (70) in Abhängigkeit von den Phasendifferenzen ($\Delta\varphi_1$, $\Delta\varphi_2$, $\Delta\varphi_3$, ...) die Manövriervorrichtung (80) zur Beeinflussung der Position (x,y,z) des medizinischen Gerätes (10) regelt,

   **dadurch gekennzeichnet, dass**

   - die Manövriervorrichtung (80) eine Spulenanordnung zur berührungslosen Führung des medizinischen Gerätes (10) ist,
   - die Manövriervorrichtung (80) zylinderförmig ausgebildet ist und
   - die Empfangseinrichtungen (11-14) in einem Innenraum der zylinderförmigen Manövriervorrichtung (80) angebracht sind.

2. Vorrichtung nach Anspruch 1, **dadurch gekennzeichnet, dass** die Signalverarbeitungseinrichtung (20) eine oder mehrere Einrichtungen (21-24) zum Bestimmen von Phasenabweichungen ($d\varphi_{11}$, $d\varphi_{12}$, $d\varphi_{13}$, $d\varphi_{14}$,...) zwischen dem Referenzsignal (R) und den empfangenen Signalen (SE11-SE14) sowie eine Differenzbildungsvorrichtung (50) zum Ermitteln der Phasendifferenzen ($\Delta\varphi_1$, $\Delta\varphi_2$, $\Delta\varphi_3$, ... ) aus den Phasenabweichungen ($d\varphi_{11}$, $d\varphi_{12}$, $d\varphi_{13}$, $d\varphi_{14}$.... ) umfasst.

3. Vorrichtung nach Anspruch 2, **dadurch gekennzeichnet, dass** jede Einrichtung (21-24) einen ersten und einen zweiten Signaleingang und einen Signalausgang aufweist, wobei

   - die Referenzsignalquelle (60) mit dem ersten Signaleingang jeder Einrichtung (21-24) verbunden ist,
   - die Empfangseinrichtungen (11-14) jeweils mit dem zweiten Signaleingang der Einrichtungen (21-24) verbunden sind,
   - die Signalausgänge der Einrichtungen (21-24) mit der Differenzbildungsvorrichtung (50) verbunden sind und
   - die Differenzbildungsvorrichtung (50) zur Übertragung der Phasendifferenzen ($\Delta\varphi_1$, $\Delta\varphi_2$, $\Delta\varphi_3$, ...) mit der Regeleinrichtung (70) verbunden ist.

4. Vorrichtung nach Anspruch 2 oder 3, **dadurch gekennzeichnet, dass** die Anzahl der Einrichtungen (21-24) der Anzahl der Empfangseinrichtungen (11-14) entspricht.

5. Vorrichtung nach Anspruch 3 oder 4, **dadurch gekennzeichnet, dass** die Einrichtungen (21-24) jeweils einen Mischer (31-34) sowie einen Phasenmesser (41-44) umfassen, wobei

   - der erste und der zweite Signaleingang an den Mischer (31-34) angeschlossen sind,
   - der Phasenmesser (41-44) an den Ausgang des Mischers angeschlossen ist und
   - der Ausgang des Phasenmessers (41-44) mit der Differenzbildungsvorrichtung (50) verbunden ist.

6. Vorrichtung zur Kontrolle einer Position (x,y,z) eines medizinischen Gerätes (10) in einem Arbeitsraum (A), wobei das medizinische Gerät (10) eine Sendeeinrichtung zum Senden eines Signals (S) umfasst, mit

- mindestens zwei Empfangseinrichtungen (11-14) zum Empfangen des Signals (S) umfassend eine erste Empfangseinrichtung (14) sowie eine oder mehrere zweite Empfangseinrichtungen (11-13) und
- einer Signalverarbeitungseinrichtung (20) zum Ermitteln von Phasendifferenzen ($\Delta\varphi_1$, $\Delta\varphi_2$, $\Delta\varphi_3$, ...) basierend auf den an den Empfangseinrichtungen (11-14) empfangenen Signalen (SE11-SE14),

wobei

- die Empfangseinrichtungen (11-14) zum Übertragen der empfangenen Signale (SE11-SE14) mit der Signalverarbeitungseinrichtung (20) verbunden sind,
- einer Regeleinrichtung (70) und
- einer Manövriervorrichtung (80), wobei
- die Signalverarbeitungseinrichtung (20) zum Übertragen der ermittelten Phasendifferenzen ($\Delta\varphi_1$, $\Delta\varphi_2$, $\Delta\varphi_3$, ...) mit einer Regeleinrichtung (70) verbunden ist und
- die Regeleinrichtung (70) in Abhängigkeit von den Phasendifferenzen ($\Delta\varphi_1$, $\Delta\varphi_2$, $\Delta\varphi_3$, ...) die Manövriervorrichtung (80) zur Beeinflussung der Position (x,y,z) des medizinischen Gerätes (10) regelt,

**dadurch gekennzeichnet, dass**

- die Manövriervorrichtung (80) eine Spulenanordnung zur berührungslosen Führung des medizinischen Gerätes (10) ist,
- die Manövriervorrichtung (80) zylinderförmig ausgebildet ist und
- die Empfangseinrichtungen (11-14) in einem Innenraum der zylinderförmigen Manövriervorrichtung (80) angebracht sind.

7. Vorrichtung nach Anspruch 6, **dadurch gekennzeichnet, dass** die Signalverarbeitungseinrichtung (20) eine oder mehrere Einrichtungen (21-23), insbesondere Phasendetektoren (21-23), zum Ermitteln der Phasendifferenzen ($\Delta\varphi_1$, $\Delta\varphi_2$, $\Delta\varphi_3$, ...) beinhaltet, wobei die Anzahl der Einrichtungen (21-23) mindestens der Anzahl der zweiten Empfangseinrichtungen (11-13) entspricht.

8. Vorrichtung nach Anspruch 7, **dadurch gekennzeichnet, dass** jede Einrichtung (21-23) einen ersten und einen zweiten Signaleingang sowie einen Signalausgang aufweist, wobei

- die erste Empfangseinrichtung (14) zum Übertragen des empfangenen Signals (SE14) mit dem ersten Signaleingang jeder Einrichtung (21-23) verbunden ist,
- die zweiten Empfangseinrichtungen (11-13) jeweils mit dem zweiten Signaleingang der Einrichtungen (21-23) verbunden sind und
- die Signalausgänge der Einrichtungen (21-23) zum Übertragen der ermittelten Phasendifferenzen ($\Delta\varphi_1$, $\Delta\varphi_2$, $\Delta\varphi_3$, ...) mit der Regeleinrichtung (70) verbunden sind.

9. Vorrichtung nach einem der Ansprüche 1 bis 8, **dadurch gekennzeichnet, dass** das medizinische Gerät (10) eine magnetisch und berührungslos navigierbare Endoskopiekapsel ist.

**Claims**

1. Apparatus for controlling a position (x,y,z) of a medical device (10) in a workspace (A), with the medical device (10) comprising a transmit facility for emitting a signal (S), having

- one or several receiving devices (11-14) for receiving the signal (S),
- a reference signal source (60) for generating a reference signal (R) and
- a signal processing facility (20) for determining phase differences ($\Delta\varphi_1$, $\Delta\varphi_2$, $\Delta\varphi_3$, ...) based on the signals (SE11-SE14) received at the receiving devices (11-14) and the reference signal (R),

with

- the receiving devices (11-14) and the reference signal source (60) for transmitting the received signals (SE11-SE14) and the reference signal (R) being connected to the signal processing facility (20),
- a control facility (70) and

- a manoeuvring apparatus (80), with
- the signal processing facility (20) for transmitting the phase differences ($\Delta\varphi_1$, $\Delta\varphi_2$, $\Delta\varphi_3$,...) being connected to the control facility (70) and
- the control facility (70) controlling the manoeuvring apparatus (80) for influencing the position (x,y,z) of the medical device (10) as a function of the phase differences ($\Delta\varphi_1$, $\Delta\varphi_2$, $\Delta\varphi_3$,...),

**characterised in that**

- the manoeuvring apparatus (80) is a coil arrangement for the contactless guidance of the medical device (10),
- the manoeuvring apparatus (80) is embodied cylindrical and
- the receiving devices (11-14) are accommodated in an interior of the cylindrical manoeuvring apparatus (80).

2. Apparatus according to claim 1, **characterised in that** the signal processing facility (20) includes one or several facilities (21-24) for determining phase deviations ($d\varphi_{11}$,$d\varphi_{12}$,$d\varphi_{13}$,$d\varphi_{14}$,...) between the reference signal (R) and the received signals (SE11-SE14) and a difference formation apparatus (50) for determining the phase differences ($\Delta\varphi_1$, $\Delta\varphi_2$, $\Delta\varphi_3$, ...) from the phase deviations ($d\varphi_{11}$, $d\varphi_{12}$,$d\varphi_{13}$,$d\varphi_{14}$,...).

3. Apparatus according to claim 2, **characterised in that** each facility (21-24) has a first and a second signal input and a signal output, with

- the reference signal source (60) being connected to the first signal input of each facility (21-24),
- the receiving devices (11-14) each being connected to the second signal input of the facilities (21-24),
- the signal outputs of the facilities (21-24) being connected to the difference formation apparatus (50) and
- the difference formation apparatus (50) being connected to the control facility (70) for transmitting the phase differences ($\Delta\varphi_1$, $\Delta\varphi_2$, $\Delta\varphi_3$, ...).

4. Apparatus according to claim 2 or 3, **characterised in that** the number of facilities (21-24) corresponds to the number of receiving devices (11-14).

5. Apparatus according to claim 3 or 4, **characterised in that** the facilities (21-24) each include a mixing device (31-34) and a phase measurer (41-44), with

- the first and the second signal input being connected to the mixing device (31-34),
- the phase measurer (41-44) being connected to the output of the mixing device and
- the output of the phase measurer (41-44) being connected to the difference formation apparatus (50).

6. Apparatus for controlling a position (x,y,z) of a medical device (10) in a workspace (A), with the medical device (10) comprising a transmit facility for emitting a signal (S), having

- at least two receiving devices (11-14) for receiving the signal (S) comprising a first receiving device (14) and one or several second receiving devices (11-13) and
- a signal processing facility (20) for determining phase differences ($\Delta\varphi_1$, $\Delta\varphi_2$, $\Delta\varphi_3$,...) based on the signals (SE11-SE14) received at the receiving devices (11-14),

with

- the receiving devices (11-14) being connected to the signal processing facility (20) for transmitting the received signals (SE11-SE14),
- a control facility (70) and
- a manoeuvring apparatus (80), with
- the signal processing facility (20) being connected to a control facility (70) for transmitting the determined phase differences ($\Delta\varphi_1$, $\Delta\varphi_2$, $\Delta\varphi_3$,...) and
- the control facility (70) controlling the manoeuvring apparatus (80) for influencing the position (x,y,z) of the medical device (10) as a function of the phase differences ($\Delta\varphi_1$, $\Delta\varphi_2$, $\Delta\varphi_3$,...),

**characterised in that**

- the manoeuvring apparatus (80) is a coil arrangement for the contactless guidance of the medical device (10),

- the manoeuvring apparatus (80) is embodied cylindrical and
- the receiving devices (11-14) are accommodated in an interior of the cylindrical manoeuvring apparatus (80).

7. Apparatus according to claim 6, **characterised in that** the signal processing facility (20) contains one or more facilities (21-23), in particular phase detectors (21-23) for determining the phase differences ($\Delta\varphi_1$, $\Delta\varphi_2$, $\Delta\varphi_3$ ,...), with the number of facilities (21-23) corresponding to at least the number of second receiving devices (11-13).

8. Apparatus according to claim 7, **characterised in that** each facility (21-23) has a first and a second signal input and a signal output, with

   - the first receiving device (15) being connected to the first signal input of each facility (21-23) for transmitting the received signal (SE14),
   - the second receiving devices (11-13) each being connected to the second signal input of the facilities (21-23) and
   - the signal outputs of the facilities (21-23) being connected to the control facility (70) for transmitting the determined phase differences ($\Delta\varphi_1$, $\Delta\varphi_2$, $\Delta\varphi_3$,...).

9. Apparatus according to one of claims 1 to 8, **characterised in that** the medical device (10) is a magnetically and contactlessly navigable endoscopy capsule.

**Revendications**

1. Dispositif de contrôle d'une position (x, y, z) d'un appareil médical (10) dans un espace de travail (A), l'appareil médical (10) comportant un moyen d'émission destiné à envoyer un signal (S), comprenant

   - un ou plusieurs moyens de réception (11-14) destinés à recevoir le signal (S),
   - une source de signal de référence (60) destinée à générer un signal de référence (R) et
   - un dispositif de traitement de signal (20) destiné à déterminer les différences de phase ($\Delta\varphi$1, $\Delta\varphi_2$, $\Delta\varphi_3$, ...) sur la base des signaux (SE11-SE14) reçus sur les moyens de réception (11-14) et du signal de référence (R),
   - les moyens de réception (11-14) et la source de signal de référence (60) étant reliés au dispositif de traitement de signal (20) pour transmettre les signaux reçus (SE11-SE14) et le signal de référence (R),
   - un moyen de réglage (70) et
   - un moyen de manoeuvre (80),
   - le dispositif de traitement de signal (20) étant relié au moyen de réglage (70) pour transmettre les différences de phase ($\Delta\varphi$1, $\Delta\varphi_2$, $\Delta\varphi_3$, ...) et
   - le moyen de réglage (70) ajustant le moyen de manoeuvre (80) en fonction des différences de phase ($\Delta\varphi$1, $\Delta\varphi_2$, $\Delta\varphi_3$, ...) pour agir sur la position (x, y, z) de l'appareil médical (10), **caractérisé en ce que**
   - le moyen de manoeuvre (80) est un ensemble de bobines servant au guidage sans contact de l'appareil médical (10),
   - le moyen de manoeuvre (80) est de forme cylindrique et
   - les moyens de réception (11-14) sont montés dans un espace intérieur du moyen de manoeuvre (80) de forme cylindrique.

2. Dispositif selon la revendication 1, **caractérisé en ce que** le dispositif de traitement de signal (20) comprend un ou plusieurs dispositifs (21-24) pour déterminer les différences de phase (d$\varphi$11, d$\varphi_{12}$, d$\varphi_{13}$, d$\varphi_{14}$, ...) entre le signal de référence (R) et les signaux reçus (SE11-SE14) ainsi qu'un dispositif de soustraction (50) pour déterminer les différences de phase ($\Delta\varphi$1, $\Delta\varphi_2$, $\Delta\varphi_3$, ...) à partir des différences de phase (d$\varphi$11, d$\varphi_{12}$, d$\varphi_{13}$, d$\varphi_{14}$, ...).

3. Dispositif selon la revendication 2, **caractérisé en ce que** chaque dispositif (21-24) comporte une première et une deuxième entrée de signal et une sortie de signal,

   - la source de signal de référence (60) étant reliée à la première entrée de signal de chaque dispositif (21-24),
   - les moyens de réception (11-14) étant reliés chacun à la deuxième entrée de signal des dispositifs (21-24),
   - les sorties de signal des dispositifs (21-24) étant reliées au dispositif de soustraction (50) et
   - le dispositif de soustraction (50) étant relié au moyen de réglage (70) pour transmettre les différences de phase ($\Delta\varphi$1, $\Delta\varphi_2$, $\Delta\varphi_3$, ...).

4. Dispositif selon la revendication 2 ou 3, **caractérisé en ce que** le nombre des dispositifs (21-24) correspond au

# EP 2 229 098 B1

nombre des moyens de réception (11-14).

5. Dispositif selon la revendication 3 ou 4, **caractérisé en ce que** les dispositifs (21-24) comprennent chacun un mélangeur (31-34) ainsi qu'un phasemètre (41-44),

- la première et la deuxième entrée de signal étant couplées au mélangeur (31-34),
- le phasemètre (41-44) étant couplé à la sortie du mélangeur et
- la sortie du phasemètre (41-44) étant reliée au dispositif de soustraction (50).

6. Dispositif de contrôle d'une position (x, y, z) d'un appareil médical (10) dans un espace de travail (A), l'appareil médical (10) comportant un moyen d'émission destiné à envoyer un signal (S), comprenant

- au moins deux moyens de réception (11-14) destinés à recevoir le signal (S), incluant un premier moyen de réception (14) ainsi qu'un ou plusieurs deuxièmes moyens de réception (11-13), et
- un dispositif de traitement de signal (20) destiné à déterminer les différences de phase ($\Delta\varphi 1$, $\Delta\varphi_2$, $\Delta\varphi_3$, ...) sur la base des signaux (SE11-SE14) reçus sur les moyens de réception (11-14) ,
- les moyens de réception (11-14) étant reliés au dispositif de traitement de signal (20) pour transmettre les signaux reçus (SE11-SE14),
- un moyen de réglage (70) et
- un moyen de manoeuvre (80),
- le dispositif de traitement de signal (20) étant relié au moyen de réglage (70) pour transmettre les différences de phase déterminées ($\Delta\varphi 1$, $\Delta\varphi_2$, $\Delta\varphi_3$, ...) et
- le moyen de réglage (70) assurant le réglage du moyen de manoeuvre (80) en fonction des différences de phase ($\Delta\varphi 1$, $\Delta\varphi_2$, $\Delta\varphi_3$, ...) pour agir sur la position (x, y, z) de l'appareil médical (10),

**caractérisé en ce que**

- le moyen de manoeuvre (80) est un ensemble de bobines servant au guidage sans contact de l'appareil médical (10),
- le moyen de manoeuvre (80) est de forme cylindrique et
- les moyens de réception (11-14) sont montés dans un espace intérieur du moyen de manoeuvre (80) de forme cylindrique.

7. Dispositif selon la revendication 6, **caractérisé en ce que** le dispositif de traitement de signal (20) comprend un ou plusieurs dispositifs (21-23), notamment des détecteurs de phase (21-23), pour déterminer les différences de phase ($\Delta\varphi 1$, $\Delta\varphi_2$, $\Delta\varphi_3$, ...) , le nombre des dispositifs (21-23) correspondant au moins au nombre des deuxièmes moyens de réception (11-13).

8. Dispositif selon la revendication 7, **caractérisé en ce que** chaque dispositif (21-23) comporte une première et une deuxième entrée de signal ainsi qu'une sortie de signal,

- le premier moyen de réception (14) étant relié à la première entrée de signal de chaque dispositif (21-23) pour transmettre le signal reçu (SE14),
- les deuxièmes moyens de réception (11-13) étant reliés chacun à la deuxième entrée de signal des dispositifs (21-23) et
- les sorties de signal des dispositifs (21-23) étant reliées au moyen de réglage (70) pour transmettre les différences de phase déterminées ($\Delta\varphi 1$, $\Delta\varphi_2$, $\Delta\varphi_3$, ...) .

9. Dispositif selon l'une des revendications 1 à 8, **caractérisé en ce que** l'appareil médical (10) est une capsule endoscopique capable d'être guidée magnétiquement et sans contact.

# FIG 1

EP 2 229 098 B1

# FIG 2

EP 2 229 098 B1

## FIG 3

## FIG 4

FIG 5

80

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10340925 B3
- WO 2005120345 A2
- EP 1440659 A2
- DE 102006019415 A1